# EUROPÄISCHE PATENTSCHRIFT

(11) **EP 4 137 626 B1**
(45) Veröffentlichungstag und Bekanntmachung des Hinweises auf die Patenterteilung: **09.07.2025**
(21) Anmeldenummer: 22195608.9
(22) Anmeldetag: 20.03.2019
(51) Int. Cl.: D04B 1/26, D04B 1/28

(54) **VERFAHREN ZUR HERSTELLUNG EINES NAHTLOSEN KOMPRESSIONSARTIKELS AUF EINER FLACHSTRICKMASCHINE**
METHOD OF MANUFACTURING A SEAMLESS COMPRESSION ARTICLE ON A FLAT KNITTING MACHINE
PROCÉDÉ DE FABRICATION D'UN ARTICLE DE COMPRESSION SANS COUTURE SUR UN MÉTIER À TRICOTER RECTILIGNE

(30) Priorität: 17.05.2018 DE 202018102766 U
(43) Veröffentlichungstag der Anmeldung: 22.02.2023
(62) Teilanmeldung aus: 19164052.3
(73) Patentinhaber: Julius Zorn GmbH, 86551 Aichach (DE)
(72) Erfinder: Lechner, Siegfried, 86529 Schrobenhausen (DE)
(74) Vertreter: Charrier Rapp & Liebau

(56) Entgegenhaltungen:
- EP-A1- 1 679 012
- EP-A2- 0 905 298
- WO-A1-2017/199520
- US-A- 2 220 803
- US-A- 4 172 456

## Beschreibung

Die Erfindung betrifft ein Verfahren zur Herstellung eines nahtlosen Kompressionsartikels auf einer Flachstrickmaschine nach dem Oberbegriff des Anspruchs 1.

Die WO 2017/199520 A1 offenbart einen auf einer Flachstrickmaschine gestrickten Kompressionsartikel, der in der Lage ist, einen Teil eines menschlichen Körpers zu komprimieren, und als zylindrischer Stützverband für die unteren Gliedmaßen, Ärmel, Socken, Strumpfhosen, Ellbogenstützverband, Hand, Handgelenkstützverband usw. ausgebildet ist. Der zylindrische Stützverband kann zur Vorbeugung und Behandlung von Krankheiten wie venösen Refluxstörungen und Lymphödemen eingesetzt werden und kann in einer Ausführungsform eine Aufnahme für Zehen des menschlichen Körpers enthalten. Entsprechend einer Veränderung des Querschnitts eines zu tragenden Körperteils wird der Umfang des gestrickten Stützverbands verändert, indem die Anzahl der Stiche in einer einzigen ringförmigen Maschenreihe auf der Maschenseite, wo das die Masche bildende Garn fortlaufend ist, verändert wird. Der als zylindrische Bandage ausgebildete Stützverband weist eine innere Oberflächenseite auf, die mit dem zu tragenden Teil des menschlichen Körpers in Kontakt zu bringen ist, wobei diese Seite in einer konkav-konvexen Form ausgebildet ist, in der konkave Abschnitte und konvexe Abschnitte abwechselnd in der Umfangsrichtung durch Stricken fortgesetzt werden.

Aus der EP 0 905 298 A2 ist ein nahtlos auf einer Flachstrickmaschine hergestelltes Schlauch-Rund-Gestrick und eine daraus gefertigte Strumpfhose mit Stützfunktion bekannt, wobei die Strumpfhose eine Fußkappe für die Zehen eines Trägers aufweist.

Die US 2 220 803 B zeigt ein auf einer Rundstrickmaschine hergestelltes Gestrick mit einer Scheinrippstruktur im Bundbereich und einem im Bereich der Scheinrippen in das Gestrick eingebundenen Elastikgarn.

Aus der EP 1 679 012 B1 ist ein nahtloser Kompressionshandschuh bekannt, der auf einer Flachstrickmaschine mit einem vorderen und einem hinteren Nadelbett gestrickt wird. Der Kompressionshandschuh besteht aus einem Grundgestrick, das entweder von einer Fingerspitze zur Öffnung des Handschuhs oder von der Öffnung zu der Fingerspitze gestrickt wird, wobei das Grundgestrick auf einer Rippstrick-Strukturbasis unter Verwendung eines Stretch-Elastikgarns, welches in einem gespannten Zustand in das Grundgestrick eingelegt wird, gestrickt ist. Bevorzugt erfolgt das Stricken von der Fingerspitze zur Öffnung hin, wobei das Stretch-Elastikgarn wenigstens an einer Stelle, an der das Stricken des Fingers beginnt, sowie an einem Fingergabelungsteil geknotet wird, um einen Abwurf des Stretch-Elastikgarns zu verhindern. Dabei wird ein vorderer Teil und ein Rückteil jedes Fingers durch ein Rippstricken hoher Maschendichte gestrickt, so dass an jedem Fingergabelungsteil ein flacher Zwickel gebildet wird. Der bekannte Kompressionshandschuh zeichnet sich durch eine hohe Stützwirkung (d.h., durch einen hohen Kompressionsdruck, den der Handschuh auf die Hand eines Trägers ausübt) aus, und kann daher für die Kompressionstherapie, beispielsweise für die Behandlung von Lymphinsuffizienzen an der Hand, eingesetzt werden.

Aufgrund seiner hohen Stützleitung, die im Wesentlichen durch das in das Grundgestrick eingebundene Strech-Elastikgarn bewirkt wird, ist der bekannte Kompressionshandschuh schwer anzuziehen. Insbesondere im Bereich der Finger ist es für einen an einer Lymphinsuffizienz leidenden Patienten schwierig, den engen, komprimierenden Handschuh über die Finger zu ziehen.

Hiervon ausgehend liegt der Erfindung die Aufgabe zugrunde, ein Verfahren zur Herstellung eines nahtlosen Kompressionsartikels, wie z.B. einen Kompressionshandschuh, einen Kompressionsstrumpf oder einen Kompressionsfüßling, aufzuzeigen, in dem wenigstens eine schlauch- oder taschenförmige Aufnahme für wenigstens einen Finger oder wenigstens einen Zeh eines Trägers des Kompressionsartikels so eingestrickt wird, dass sich der gestrickte Kompressionsartikel einfacher anziehen lässt und dennoch eine hohe Stützleistung aufweist und einen hohen und gleichmäßigen Kompressionsdruck auf eine Körperextremität des Trägers, an der der Kompressionsartikel angelegt wird, ausüben kann.

Diese Aufgabe wird mit dem Verfahren mit den Merkmalen des Anspruchs 1 gelöst. Bevorzugte Ausführungsformen des Verfahrens sind in den abhängigen Ansprüchen aufgezeigt.

Der Kompressionsartikel wird in dem erfindungsgemäßen Verfahren nahtlos auf einer Flachstrickmaschine mit einem vorderen und einem hinteren Nadelbett gestrickt und weist wenigstens eine schlauch- oder taschenförmige und sich entlang einer Längsrichtung erstreckende Aufnahme für wenigstens einen Finger oder wenigstens einen Zeh eines Trägers des Kompressionsartikels auf und ist an einer sich zumindest im Wesentlichen in der Längsrichtung erstreckenden Körperextremität des Trägers, beispielsweise eine Hand- oder einen Fuß, anlegbar. Das Anziehen des Kompressionsartikels wird dadurch erleichtert, dass in dem Verfahren zur Herstellung des Kompressionsartikels in die oder jede Aufnahme eine Mehrzahl von rippenförmigen Erhebungen, die zumindest im Wesentlichen parallel zueinander und entlang der Längsrichtung der jeweiligen Aufnahme verlaufen, eingestrickt werden.

Es hat sich gezeigt, dass die bekannten nahtlosen Kompressionsartikel, insbesondere der eingangs beschriebene Kompressionshandschuh, die auf einer Flachstrickmaschine unter Einbindung eines elastischen Schussfadens in ein Grundgestrick gestrickt werden, deshalb schwierig anzuziehen sind, weil sie eine hohe Längszügigkeit, d.h., eine hohe Dehnbarkeit in Längsrichtung, aufweisen. Die Längszügigkeit wird dabei einerseits durch die Ausbildung des Grundgestricks und andererseits durch die Verwendung eines elastischen Strickfadens, aus dem das Grundgestrick gestrickt wird, hervorgerufen. Die Verwendung eines elastischen Strickfadens ist für die Erzeugung einer hohen Stützleistung bzw. einer hohen Kompressionswirkung zweckdienlich.

Der Erfindung liegt die Erkenntnis zugrunde, dass die hohe Längszügigkeit der bekannten, nahtlos auf einer Flachstrickmaschine gestrickten Kompressionsartikel reduziert werden kann, wenn zumindest im Bereich der oder jeder Aufnahme des Kompressionsartikels, die zur Aufnahme wenigstens eines Fingers oder Zehs des Trägers vorgesehen ist, eine Mehrzahl von rippenförmigen Erhebungen, die parallel zueinander verlaufen und sich entlang der Längsrichtung der jeweiligen Aufnahme erstrecken, in das Grundgestrick eingestrickt werden. Die rippenförmigen Erhebungen stehen dabei auf der Außenseite über dem Grundgestrick vor und kommen auf der Innenseite nicht zum Vorschein. Die rippenförmigen Erhebungen, die auf der Außenseite über dem Grundgestrick vorstehen, sind dabei zu unterscheiden von den Rippen, die sich beim Stricken des Grundgestricks auf Basis einer Rippstricktechnik bzw. eines Rippenstrickmusters ergeben und sich über die gesamte Fläche des in Form eines Rippenstrickmusters gestrickten Grundgestricks erstrecken. Das Grundgestrick des erfindungsgemäß hergestellten Kompressionsartikels kann demgemäß ebenfalls mit einer Rippstrick-Technik bzw. mit einem Rippenstrickmuster gestrickt werden, weist dann allerdings zusätzlich zu dem sich durch die Rippstricktechnik ausbildenden Rippenmuster des Grundgestricks, das sich über die gesamte Oberfläche des Grundgestricks erstreckt, zusätzlich noch im Bereich der oder jeder Aufnahme für wenigstens einen Finger oder Zeh eine Mehrzahl von rippenförmigen Erhebungen auf, die an der Außenseite über dem Grundgestrick vorstehen.

Der erfindungsgemäß hergestellte Kompressionsartikel besteht zweckmäßig aus einem Grundgestrick mit einer beim Tragen an einer Körperextremität dieser zugewandten Innenseite und einer der Innenseite gegenüberliegenden Außenseite. Das Grundgestrick wird dabei entlang einer Maschenstäbchenrichtung gestrickt und weist quer zur Maschenstäbchenrichtung verlaufende Maschenreihen auf. Beim Anlegen des erfindungsgemäß hergestellten Kompressionsartikels an eine Körperextremität eines Trägers verläuft dabei die Maschenstäbchenrichtung zumindest im Wesentlichen entlang der Längsrichtung der Körperextremität. Insbesondere verläuft die Maschenstäbchenrichtung im Bereich der oder jeder Aufnahme des Kompressionsartikels, der zur Aufnahme wenigstens eines Fingers oder Zehs des Trägers vorgesehen ist, entlang der Längsrichtung der jeweiligen Aufnahme.

Eine Aufnahme kann dabei, je nach Ausdehnung der Aufnahme quer zur Längsrichtung, zur Aufnahme nur eines Fingers oder Zehs oder auch zur Aufnahme mehrerer Finger bzw. Zehen vorgesehen sein.

Die Ausbildung der rippenförmigen Erhebungen in der Weise, dass Sie an der Außenseite des Kompressionsartikels über dem Grundgestrick vorstehen, erleichtert zum einen die Konfektionierung des Kompressionsartikels auf einer Flachstrickmaschine und verhindert außerdem, dass ein Träger des Kompressionsartikels beim Anziehen mit einem Fingernagel oder einem Zehennagel an den rippenförmigen Erhebungen hängen bleiben könnte.

Es hat sich gezeigt, dass die Anziehbarkeit des erfindungsgemäß hergestellten Kompressionsartikels bereits durch Ausbildung von wenigstens zwei rippenförmigen Erhebungen an jeder schlauch- oder taschenförmigen Aufnahme verbessert werden kann. Je nach Umfang der schlauch- oder taschenförmigen Aufnahmen können bis zu 30 rippenförmige Erhebungen an jeder Aufnahme eingestrickt werden. Bevorzugt weist jede Aufnahme zwischen 3 und 7 rippenförmige Erhebungen auf.

Die rippenförmigen Erhebungen erstrecken sich in jeder Maschenreihe des Grundgestricks bevorzugt über mindestens drei Maschen.

Zur Erzielung einer hohen Kompressionswirkung ist es vorteilhaft, wenn in das aus einem Strickfaden gestrickte Grundgestrick ein elastischer Schussfaden eingelegt bzw. eingebunden wird. Eine besonders hohe Kompressionswirkung kann dabei erzielt werden, wenn in jeder Maschenreihe des Grundgestricks ein elastischer Schussfaden eingelegt bzw. eingebunden wird.

Die sich entlang der Längsrichtung der jeweiligen Aufnahme für einen Finger oder einen Zeh erstreckenden Erhebungen sind in einem in Maschenreihenrichtung liegenden, vorgegebenen Abstand zueinander in dem Grundgestrick angeordnet. Bevorzugt liegen zwischen zwei in einer Maschenreihe aufeinanderfolgende Erhebungen wenigstens zwei Maschen des Strickfadens, aus dem das Grundgestrick gestrickt ist. Der Abstand zwischen benachbarten Erhebungen in einer Maschenreihe kann sich jedoch auch über mehr als zwei Maschen des Grundgestricks, bspw. über fünf bis zehn Maschen, erstrecken.

Wenn eine geringere Kompressionswirkung benötigt wird, kann auch nur in jeder zweiten Maschenreihe ein elastischer Schussfaden vorgesehen sein. Weiterhin ist es möglich, in druckgeminderten Bereichen des Grundgestricks, die sich über mehr als zwei in Maschenstäbchenrichtung aufeinanderfolgende Maschenreihen erstrecken, in mehreren, aufeinander folgenden Maschenreihen keinen Schussfaden einzubringen.

Die Ausbildung der rippenförmigen Erhebungen in dem Grundgestrick erfolgt zweckmäßig durch Flottungen des Strickfadens des Grundgestricks und/oder durch Ausbildung von Fanghenkeln. Dabei kann in einem bevorzugten Ausführungsbeispiel des Kompressionsartikels, in dem in das Grundgestrick ein Schussfaden eingebunden wird, der Schussfaden zwischen zwei in Richtung der Maschenreihen aufeinanderfolgende Erhebungen als Fang in das Grundgestrick eingebunden werden. Bevorzugt wird der Schussfaden dabei zwischen zwei in Maschenreihenrichtung aufeinanderfolgende Erhebungen in jeder zweiten Masche des Grundgestricks als Fang in das Grundgestrick eingebunden.

Eine Abgrenzung der über das Grundgestrick vorstehenden Erhebungen kann durch Ausbildung von zwei in den Maschenreihen aufeinanderfolgenden Fangmaschen des Schussfadens in den Randbereichen der Erhebungen ausgebildet werden.

Zur Minimierung der Längszügigkeit der zur Aufnahme wenigstens eines Fingers oder Zehs vorgesehenen Aufnahmen des Kompressionsartikels ist es zweckmäßig, wenn sich die Erhebungen in Längsrichtung der oder jeder Aufnahme zumindest im Wesentlichen über die gesamte Ausdehnung der Aufnahme in Längsrichtung erstrecken.

Um eine möglichst hohe Kompressionswirkung des Kompressionsartikels außerhalb des Bereichs, in dem sich die Aufnahmen befinden, zu gewährleisten, ist es zweckmäßig, wenn in den Bereichen außerhalb der Aufnahmen keine rippenförmigen Erhebungen im Sinne der Erfindung eingestrickt werden. Insbesondere bei Maßanfertigungen von Kompressionsartikeln gemäß der Erfindung ist es darüber hinaus einfacher, den Kompressionsartikel zu konfektionieren, wenn außerhalb der Aufnahmen keine rippenförmigen Erhebungen vorhanden sind.

Der Kompressionsartikel wird erfindungsgemäß auf einer Flachstrickmaschine mit einem vorderen und einem hinteren Nadelbett gestrickt, wobei das Grundgestrick des Kompressionsartikels dadurch eine vordere Lage, die auf dem vorderen Nadelbett gestrickt wird, und eine hintere Lage, die auf dem hinteren Nadelbett gestrickt wird, erhält und dadurch schlauchförmig ausgebildet wird. Die vordere und die hintere Lage des Kompressionsartikels werden dabei nahtlos miteinander verbunden, d.h. miteinander verstrickt. Dabei enthält die vordere und die hintere Lage des Grundgestricks jeweils einen Strickfaden, wobei der Strickfaden zur Ausbildung der rippenförmigen Erhebungen Flottungen bildet und die Stellen, an denen der Strickfaden flott liegt, werden in jeweils in Maschenstäbchenrichtung aufeinanderfolgenden Maschenreihen um wenigstens eine Masche, bevorzugt um eine oder zwei Maschen, versetzt zueinander angeordnet. Durch die Anzahl der Maschen, um die die Versetzung der Flottungen des Strickfadens erfolgt, kann die Breite der rippenförmigen Erhebungen (also deren Ausdehnung in Maschenreihenrichtung) definiert werden.

Diese und weitere Vorteile sowie zweckmäßige Merkmale der Erfindung ergeben sich aus den nachfolgend unter Bezugnahme auf die begleitenden Zeichnungen näher beschriebenen Ausführungsbespielen. Die Zeichnungen zeigen:
- **Fig. 1:**: Schematische Darstellung eines Strickbilds einer ersten Ausführungsform eines erfindungsgemäß hergestellten Gestricks, aus dem zumindest ein Teilbereich eines Kompressionsartikels hergestellt werden kann;
- **Fig. 2:**: Darstellung der Außenseite (Figur 2a) und der Innenseite (Fig. 2b) eines für die erfindungsgemäße Herstellung eines Kompressionsartikels bevorzugten Gestricks;
- **Fig. 3:**: Darstellung von als Kompressionsärmel ausgebildeten Kompressionsartikeln, die gemäß der Erfindung hergestellt werden können, wobei in Figur 3a eine erste Ausführungsform eines Kompressionsärmels gezeigt ist, in dem lediglich im Bereich der Fingeraufnahmen rippenförmige Erhebungen vorgesehen sind, und in Figur 3b eine zweite Ausführungsform eines Kompressionsärmels gezeigt ist, bei dem über die gesamte Länge des Ärmels rippenförmige Erhebungen vorgesehen sind;
- **Fig. 4:**: Darstellung von als Kompressionshandschuh ausgebildeten Kompressionsartikeln, die gemäß der Erfindung hergestellt werden können, wobei in Figur 3a eine erste Ausführungsform eines Kompressionshandschuhs gezeigt ist, in dem lediglich im Bereich der Fingeraufnahmen rippenförmige Erhebungen vorgesehen sind, und in Figur 3b eine zweite Ausführungsform eines Kompressionshandschuh gezeigt ist, bei dem über die gesamte Länge des Ärmels rippenförmige Erhebungen gemäß der Erfindung vorgesehen sind;
- **Fig. 5:**: Darstellung eines als Kompressionsstrumpf (Fig. 5a) und als Füßling (Fig. 5b) ausgebildeten Ausführungsbeispiels eines Kompressionsartikels, wobei sich jeweils im Bereich der Zehenaufnahmen des Kompressionsstrumpfs der Figur 5a und des Füßlings der Figur 5b rippenförmige Erhebungen in Längsrichtung der jeweiligen Zehenaufnahme erstrecken;
- **Fig. 6:**: Schematische Darstellung eines Strickbilds einer weiteren Ausführungsform eines Gestricks, aus dem zumindest ein Teilbereich eines Kompressionsartikels erfindungsgemäß hergestellt werden kann.

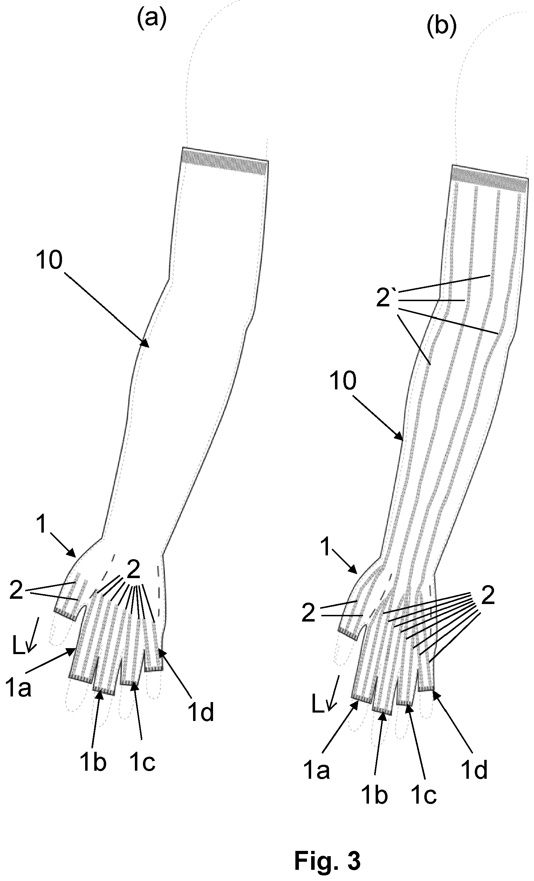

Die beispielhaft in Figur 3 dargestellten und als Kompressionsärmel ausgebildeten Ausführungsbeispiele eines Kompressionsartikels weisen fünf Aufnahmen 1, 1a, 1b, 1c, 1d zur Aufnahme der fünf Finger einer Hand eines Trägers der Kompressionsärmel auf, wobei eine Aufnahme 1 zur Aufnahme des Daumens und die anderen Aufnahmen 1a - 1d zur Aufnahme der übrigen Finger der Hand vorgesehen sind. Die Aufnahmen, die im Folgenden zusammen jeweils mit Bezugsziffer 1 gekennzeichnet sind, erstrecken sich dabei entlang einer Längsrichtung L, die der Längsrichtung des jeweiligen Fingers der Hand entspricht. Bei dem hier zeichnerisch dargestellten Ausführungsbeispiel sind die Fingeraufnahmen 1 jeweils offen und weisen am offenen Ende ein Bündchen auf. Im Rahmen der Erfindung können die Aufnahmen 1 jedoch auch geschlossen, d.h., mit einer geschlossenen Fingerspitze, ausgebildet sein. Bei dieser Ausführungsform entfällt das Bündchen.

In den beiden in Figur 3 gezeigten Ausführungsbeispielen eines Kompressionsartikels in Form eines Kompressionsärmels sind im Bereich der Aufnahmen 1 jeweils mehreren, rippenförmige Erhebungen 2 vorgesehen, die zumindest im Wesentlichen parallel zueinander verlaufen und sich entlang der Längsrichtung L der jeweiligen Aufnahme 1 erstrecken. An jeder Aufnahme 1 ist dabei eine Mehrzahl von rippenförmigen Erhebungen 2 angeordnet, wobei sich die rippenförmigen Erhebungen 2 zumindest im Wesentlichen über die gesamte Ausdehnung der jeweiligen Aufnahme 1 in ihrer Längsrichtung L erstrecken. In dem in Figur 3a gezeigten Ausführungsbeispiel eines Kompressionsärmels sind außerhalb der Bereiche der Aufnahmen 1 keine Erhebungen 2 vorgesehen. Bei dem in Figur 3b gezeigten Ausführungsbeispiel eines Kompressionsärmels sind dagegen auch in den Bereichen außerhalb der Aufnahmen 1 rippenförmige Erhebungen 2' vorgesehen, die sich zumindest im Wesentlichen entlang der Längsrichtung des Ärmels erstrecken und wenigstens annäherend parallel zueinander verlaufen.

Die rippenförmigen Erhebungen 2, 2' vermindern dabei die Längszügigkeit des Kompressionsartikels im Bereich der Aufnahmen 1 bzw. in dem gesamten Bereich des Kompressionsartikels und erleichtern dadurch das Anziehen des Kompressionsärmels, insbesondere das Überziehen der Aufnahmen 1 über die Finger der Hand des Trägers bzw. über die gesamte Hand und den Arm des Trägers.

Die in Figur 3 gezeigten Kompressionsärmel sind, wie alle anderen Ausführungsformen der Kompressionsartikel, gemäß der Erfindung nahtlos auf einer Flachstrickmaschine mit einem vorderen und einem gegenüberliegenden hinteren Nadelbett gestrickt, wobei die schlauch- oder taschenförmigen Aufnahmen 1, die zur Aufnahme wenigstens eines Fingers oder eines Zehs des Trägers vorgesehen sind und sich entlang der Längsrichtung L erstrecken, nahtlos in dem Kompressionsartikel eingestrickt werden.

Das Gestrick, aus dem die Kompressionsartikel mit dem erfindungsgemäßen Verfahren hergestellt werden können, umfasst ein Grundgestrick aus einem Strickfaden mit einer beim Tragen des Kompressionsartikels an einer Körperextremität dieser zugewandten Innenseite und einer der Innenseite gegenüberliegenden Außenseite, wobei das Grundgestrick entlang einer Maschenstäbchenrichtung gestrickt wird und sich quer zu Maschenstäbchenrichtung verlaufende Maschenreihen aufweist.

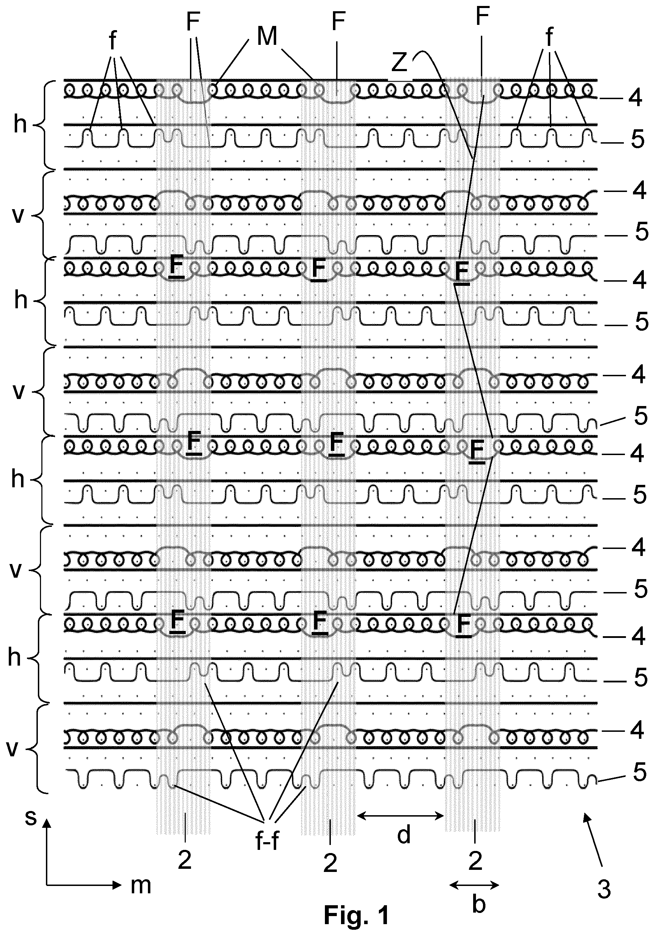

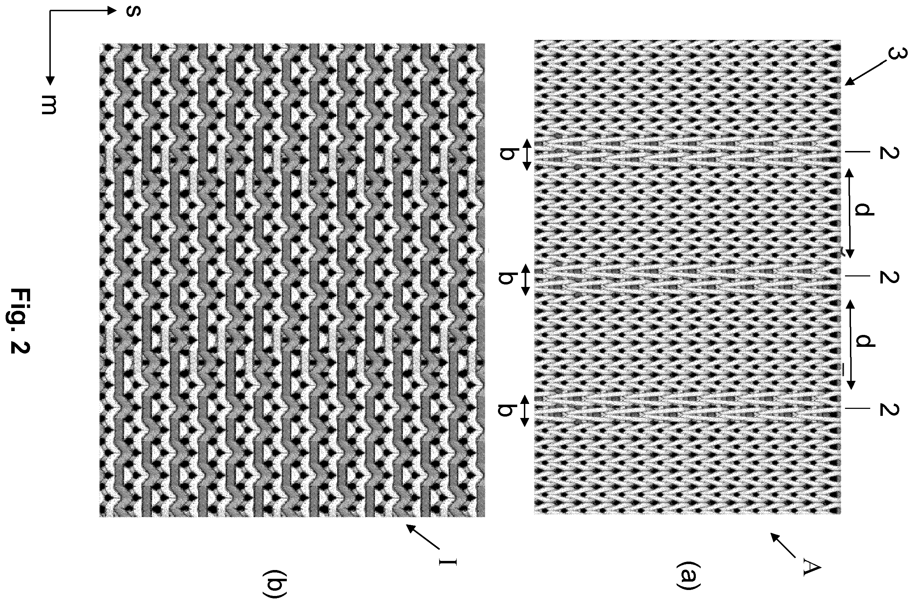

In den Figuren 1 und 2 ist ein bevorzugtes Gestrick dargestellt, aus dem zumindest die Bereiche der Aufnahmen 1 der Kompressionsartikel hergestellt werden können.

Aus dem Strickbild von Figur 1 ist ersichtlich, dass das Gestrick eine vordere Lage v, die auf dem vorderen Nadelbett der Flachstrickmaschine gestrickt wird, und eine hintere Lage h, die auf dem hinteren Nadelbett gestrickt wird, enthält. In Figur 1 sind die Maschenreihen der vorderen Lage v und der hinteren Lage h entlang der Maschenstäbchenrichtung s übereinander dargestellt. Aus Figur 1 ist die Ausbildung des Grundgestricks der vorderen Lage v und der hinteren Lage h mit den in den Maschenreihen m verlaufenden Maschen M des Grundgestricks ersichtlich.

Das Grundgestrick ist dabei aus einem Strickfaden 4 gebildet, der beispielsweise in einem Rechts-Links-Gestrick oder in einer 1:1-Bindung zur Ausbildung des Grundgestricks auf der Flachstrickmaschine verstrickt wird. In dem in Figur 1 gezeigten Ausführungsbeispiel ist das Grundgestrick als Rechts-Links-Gestrick ausgebildet. Bei dem Strickfaden 4 kann es sich um einen elastischen oder auch um einen zumindest weitgehend unelastischen Strickfaden handeln. Für die Ausbildung einer hohen Kompressionswirkung ist die Verwendung eines elastischen Strickfadens zu bevorzugen. Wenn ein elastischer Strickfaden 4 eingesetzt wird, kann es sich beispielsweise um ein Umwindegarn mit einem elastischen Kernfaden handeln.

Bei dem in Figur 1 gezeigten bevorzugten Ausführungsbeispiel eines für die erfindungsgemäße Herstellung eines Kompressionsartikels geeigneten Gestricks ist in jeder Maschenreihe m des aus dem Strickfaden 4 gebildeten Grundgestricks ein elastischer Schussfaden 5 eingebunden. Bei dem elastischen Schussfaden 5 kann es sich beispielsweise um ein Umwindegarn mit einem hochelastischen Kernfaden oder um einen Elastan- oder Gummifaden handeln. Zweckmäßig weist der Kernfaden oder der gesamte Schussfaden eine Dicke im Bereich von 200 bis 1500 dtex auf. Der Schussfaden 5 ist dabei in der Regel (wesentlich) dicker als der Strickfaden 4.

Die rippenförmigen Erhebungen 2, die zumindest im Bereich der Aufnahme 1 des Kompressionsartikels eingestrickt werden, werden durch Flottungen F des Strickfadens 4 in den Maschenreihen m des Grundgestricks ausgebildet. In dem Strickbild der Figur 1 sind die zumindest im Wesentlichen parallel zueinander und in Maschenreihenrichtung in einem vorgegebenen Abstand d zueinander angeordneten Erhebungen 2 durch ein graues Linienfeld dargestellt. Die Ausdehnung der Erhebungen 2 in Maschenreihenrichtung m, also deren Breite b, ergibt sich dabei durch die Lage der Flottungen F des Strickfadens 4, die in dem Strickbild der Figur 1 jeweils mit Bezugzeichen F dargestellt sind. In jeweils in Maschenstäbchenrichtung s aufeinanderfolgenden (bzw. übereinanderliegenden) Maschenreihen m der vorderen Lage v und der hinteren Lage a sind die Stellen, an denen der Strickfaden 4 Flottungen F bildet, um wenigstens eine Masche versetzt zueinander angeordnet. Bei dem in Figur 1 dargestellten Strickbild sind die Stellen, an denen der Strickfaden 4 in der vorderen Lage v bzw. der hinteren Lage h Flottungen F bildet, jeweils um eine Masche versetzt zueinander angeordnet. Eine Verbindung Z der Flottungsstellen F in der vorderen Lage v bzw. der hinteren Lage h bildet daher eine um jeweils eine Masche versetzte Zick-Zack-Linie und die Ränder dieser Zick-Zack-Linie in den Maschenreihen m stellen die seitlichen Begrenzungen der Erhebungen 2 dar.

In Maschenreihenrichtung m zwischen zwei aufeinanderfolgende Erhebungen 2 wird eine vorgegebene Anzahl von Maschen M des Strickfaden 4 ausgebildet. Die Anzahl der Maschen M, die sich zwischen zwei in Maschenreihenrichtung m benachbarten Erhebungen 2 befinden, legt den Abstand d der zueinander benachbarten Erhebungen 2 in Maschenreihenrichtung m fest. Bevorzugt liegen zwischen zwei in Maschenreihenrichtung benachbarten Erhebungen 2 wenigstens zwei und bevorzugt mehr als fünf Maschen M. Bei dem in Figur 1 gezeigten Ausführungsbeispiel liegen sechs Maschen M des Strickfadens 4 zwischen in Maschenreihenrichtung m benachbarten Erhebungen 2.

Wie aus dem Strickbild der Figur 1 ersichtlich, wird in jeder Maschenreihe m ein Schussfaden 5 eingebunden. Der Schussfaden 5 bildet dabei Fangmaschen f. Insbesondere in den Randbereichen der Erhebungen 2 bildet der Schussfaden in den Maschenreihen m zwei unmittelbar aufeinanderfolgende Fangmaschen f-f. In den Bereichen zwischen zwei in einer Maschenreihe benachbarten Erhebungen 2 ist der Schussfaden 5 abwechselnd als Fangmasche f und flott liegend in dem durch den Strickfaden 4 gebildeten Grundgestrick eingelegt. Durch die Ausbildung einer doppelten Fangmasche f-f im Randbereich der Erhebungen 2 wird das aus dem Strickfaden 4 gebildete Grundgestrick im Bereich der Erhebungen 2, in dem der Strickfaden 4 eine Flottung F bildet, zusammengezogen, so dass sich eine über den übrigen Bereich des Grundgestricks vorstehende Erhebung 2 ausbildet.

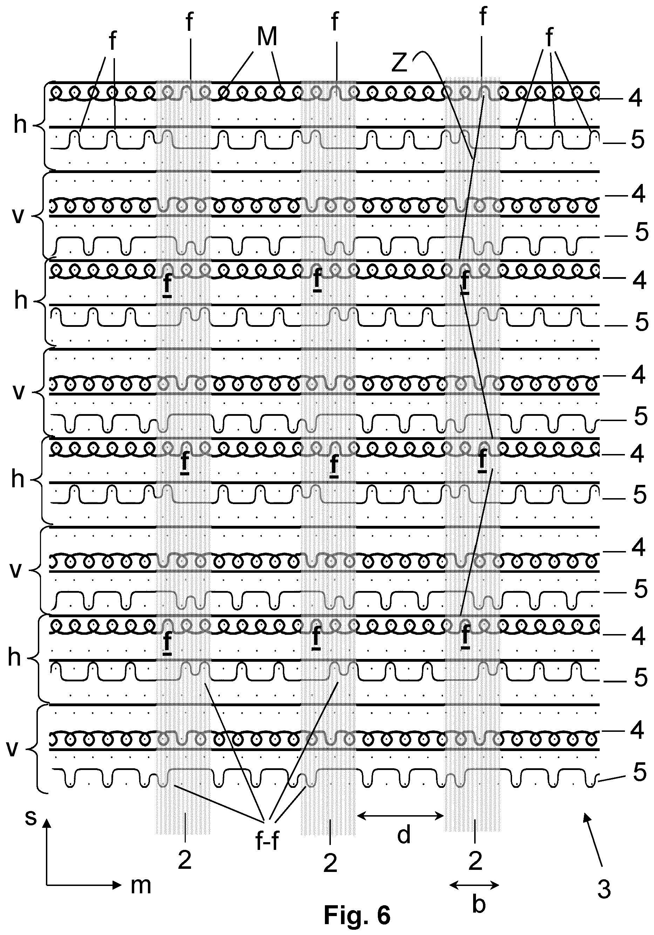

In Figur 6 ist eine weitere Ausführungsform eines Gestricks dargestellt, aus dem zumindest die Bereiche der Aufnahmen 1 der Kompressionsartikel erfindungsgemäß hergestellt werden können. Diese Ausführungsform unterscheidet sich von dem in Figur 1 gezeigten Gestrick dadurch, dass die Erhebungen 2 durch Ausbildung von Fanghenkeln f durch den Strickfaden 4 ausgebildet sind. Hierfür sind an den Stellen, an denen der Strickfaden 4 bei der Ausführungsform der Figur 1 Flottungen F bildet, an Stelle der Flottungen Fanghenkel f ausgebildet. An den Stellen der Fanghenkel f bildet der Strickfaden 4 - ebenso wie bei den Flottungsstellen F der Ausführungsform von Figur 1 - keine Maschen, wodurch die Erhebungen 2 entstehen.

Bevorzugt wird ein Kompressionsartikel aus dem in Figur 1 oder dem in Figur 6 schematisch dargestellten Gestrick in dem erfindungsgemäßen Verfahren so gestrickt, dass die über dem Grundgestrick vorstehenden rippenförmigen Erhebungen 2 auf der Außenseite des Kompressionsartikels, der beim Anlegen an einer Körperextremität dieser abgewandt ist, zu liegen kommen.

In Figur 2 ist die Außenseite A und die Innenseite I des Gestricks von Figur 1 dargestellt, wobei Figur 2a die Außenseite A und Figur 2b die Innenseite I zeigt. Aus Figur 2a sind die Erhebungen 2, die sich in Längsrichtung L und in Maschenreihenrichtung m im Abstand d zueinander erstrecken und eine Breite b aufweisen, ersichtlich. Die Erhebungen 2 stehen dabei über das Grundgestrick vor und bilden dadurch rippenförmige Erhebungen 2. Auf der Innenseite I des Gestricks kommen die Erhebungen 2 nicht zum Vorschein. Dies hat den Vorteil, dass die Erhebungen 2 beim Anziehen des Kompressionsartikels nicht stören und der Träger des Kompressionsartikels insbesondere nicht mit einem Finger- oder Zehennagel an den Erhebungen 2 hängen bleiben kann.

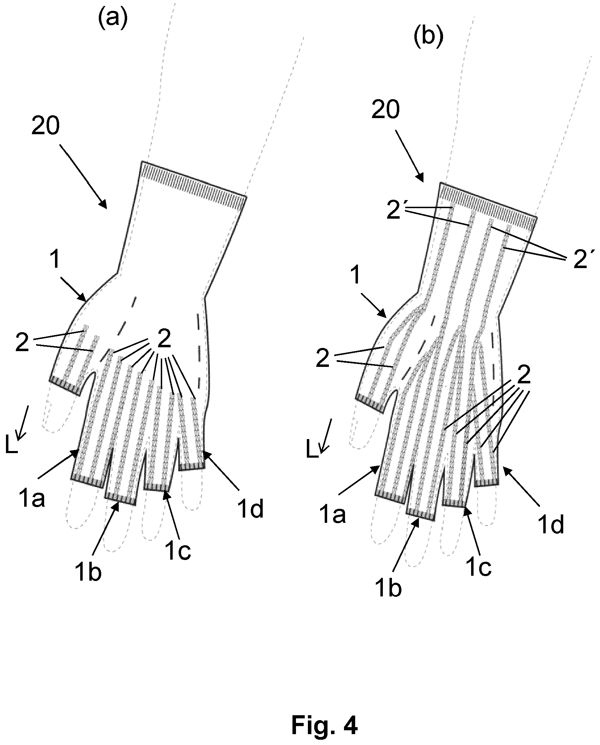

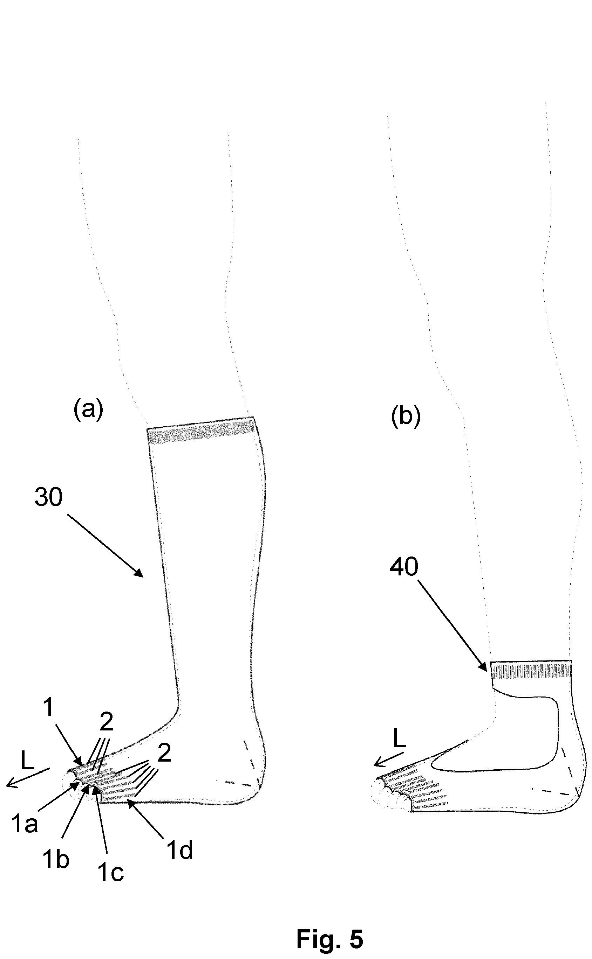

In den Figuren 4 und 5 sind weitere Ausführungsbeispiele von Kompressionsartikeln gezeigt, welche gemäß der Erfindung hergestellt werden können. Figur 4 zeigt dabei zwei als Kompressionshandschuhe ausgebildete Kompressionsartikel, wobei bei dem in Figur 4a gezeigten Kompressionshandschuh lediglich im Bereich der Aufnahmen 1 für die Finger des Trägers rippenförmige Erhebungen 2 vorgesehen sind, wohingegen bei dem in Figur 4b gezeigten Ausführungsbeispiel eines Kompressionshandschuhs auch in dem Bereich außerhalb der Aufnahmen 1 rippenförmige Erhebungen 2' angeordnet sind.

In Figur 5 sind erfindungsgemäß herstellbare Kompressionsartikel zum Anlegen an ein Bein bzw. einen Fuß eines Trägers gezeigt. In Figur 5a ist ein Kompressionsstrumpf dargestellt, der an einem Unterschenkel eines Trägers angelegt werden kann. Der Unterschenkel-Kompressionsstrumpf weist dabei Aufnahmen 1, 1a, 1b, 1c, 1d zur Aufnahme der Zehen des Trägers auf. Die Aufnahmen 1, 1a-1d sind dabei jeweils offen ausgebildet und enthalten am offenen Ende ein Bündchen. Es ist jedoch auch möglich, die Zehenaufnahmen 1, 1a-1d geschlossen auszubilden, so dass die Zehen des Trägers vollständig in der jeweils vorgesehenen Aufnahme 1, 1a-1d aufgenommen werden können. Wir in den Ausführungsführungsbeispielen der Figuren 3 und 4 sind auch bei den in Figur 5 gezeigten Ausführungsbeispielen im Bereich der Aufnahmen 1 für die Zehen rippenförmige Erhebungen 2 angeordnet, die sich jeweils in Längsrichtung L der jeweiligen Aufnahme 1 erstrecken und quer zur Längsrichtung in einem vorgegebenen Abstand d zueinander sind und parallel zueinander verlaufen. Außerhalb der Aufnahmen 1 sind dabei keine Erhebungen 2 vorgesehen. Das in Figur 5b gezeigte Ausführungsbeispiel zeigt einen Füßling mit Aufnahmen 1 zur Aufnahme der Zehen des Trägers. Der Füßling weist im Bereich des Rists und des Sprunggelenks eine Ausnehmung auf. Diese Ausnehmung kann auch weggelassen werden, so dass der Fuß des Trägers, einschl. des Rists und des Sprunggelenks, vollständig in Form einer Socke von dem Kompressionsartikel umfasst wird.

Es ist für einen Fachmann leicht ersichtlich, dass weitere Formen von Kompressionsartikeln mit wenigstens einer Aufnahme für mindestens einen Finger oder Zeh, bspw. Socken oder Fingerlinge, gemäß der Erfindung ausgebildet werden können.

## Patentansprüche

1. Verfahren zur Herstellung eines nahtlosen Kompressionsartikels auf einer Flachstrickmaschine mit einem vorderen Nadelbett und einem diesem gegenüberliegenden hinteren Nadelbett, wobei auf der Flachstrickmaschine ein Grundgestrick (3) aus wenigstens einem Strickfaden (4) und wenigstens einem darin eingelegten oder eingestrickten elastischen Schussfaden (5) gestrickt wird, wobei das Grundgestrick (3) eine beim Tragen der Körperextremität zugewandte Innenseite (I) und eine dieser gegenüber liegenden Außenseite (A) aufweist und das Grundgestrick (3) entlang einer sich zumindest im Wesentlichen in Längsrichtung (L) der oder jeder Aufnahme (1) erstreckenden Maschenstäbchenrichtung (s) gestrickt wird und quer zur Maschenstäbchenrichtung (s) verlaufende Maschenreihen (m) aufweist, und der aus dem Grundgestrick (3) gestrickte Kompressionsartikel wenigstens eine schlauch- oder taschenförmige und sich entlang einer Längsrichtung (L) erstreckende Aufnahme (1) für wenigstens einen Finger (F) oder wenigstens einen Zeh (Z) eines Trägers des Kompressionsartikels aufweist, so dass der Kompressionsartikel an einer sich in Längsrichtung (L) erstreckenden Körperextremität des Trägers anlegbar ist, wobei in jeder Aufnahme (1) eine Mehrzahl von rippenförmigen Erhebungen (2), die zumindest im Wesentlichen parallel zueinander und entlang der Längsrichtung (L) der jeweiligen Aufnahme (1) verlaufen, eingestrickt wird, wobei die rippenförmigen Erhebungen (2) an der Außenseite (A) über dem Grundgestrick (3) vorstehen, **dadurch gekennzeichnet dass** die rippenförmigen Erhebungen (2) an der Innenseite (I) nicht zum Vorschein kommen.

2. Verfahren nach Anspruch 1, **dadurch gekennzeichnet, dass** an jeder Aufnahme (1) zwischen zwei und 30, bevorzugt zwischen drei und sieben Erhebungen (2) angeordnet werden und/oder dass sich die Erhebungen (2) in einer Maschenreihe (m) des Grundgestricks (3) über mindestens zwei, bevorzugt über drei oder vier Maschen erstrecken.

3. Verfahren nach einem der voranstehenden Ansprüche, **dadurch gekennzeichnet, dass** benachbarte Erhebungen (2) parallel und in einem vorgegebenen Abstand (d) zueinander verlaufend gestrickt werden, wobei bevorzugt zwischen zwei in einer Maschenreihe (m) aufeinanderfolgende Erhebungen (2) wenigstens zwei Maschen des Strickfadens (4), bevorzugt zwischen drei und 20 Maschen, besonders bevorzugt zwischen fünf und zehn Maschen des Strickfadens (4) liegen.

4. Verfahren nach einem der voranstehenden Ansprüche, **dadurch gekennzeichnet, dass** in jeder oder in jeder zweiten Maschenreihe (m) des Grundgestricks (3) ein Schussfaden (5) eingestrickt oder eingebunden wird und dass optional in druckgeminderten Bereichen des Grundgestricks (3) mehrere in Längsrichtung (L) aufeinanderfolgende Maschenreihen (m) gestrickt werden, in denen kein Schussfaden verläuft.

5. Verfahren nach einem der voranstehenden Ansprüche, **dadurch gekennzeichnet, dass** der Schussfaden (5) zwischen zwei in Richtung der Maschenreihen (m) aufeinanderfolgende Erhebungen (2) in jeder oder jeder zweiten Masche als Fangmasche (f) in das Grundgestrick (3) des Strickfadens (4) eingebunden wird.

6. Verfahren nach einem der voranstehenden Ansprüche, **dadurch gekennzeichnet, dass** der Schussfaden (5) in einem Randbereich der Erhebungen (2) zwei in einer Maschenreihe (m) aufeinanderfolgende Fangmaschen (f-f) bildet.

7. Verfahren nach einem der voranstehenden Ansprüche, **dadurch gekennzeichnet, dass** sich die Erhebungen (2) in Längsrichtung (L) der oder jeder Aufnahme (1) zumindest im Wesentlichen über die gesamte Ausdehnung der jeweiligen Aufnahme (1) erstrecken, wobei bevorzugt in den Bereichen des Kompressionsartikels außerhalb der Aufnahmen (1) keine Erhebungen (2) gestrickt werden.

8. Verfahren nach einem der voranstehenden Ansprüche, **dadurch gekennzeichnet, dass** die rippenförmigen Erhebungen (2) durch Flottungen (F) und/oder Fangmaschen (f) des Strickfadens (3) gebildet werden.

9. Verfahren nach einem der voranstehenden Ansprüche, **dadurch gekennzeichnet, dass** die Flachstrickmaschine ein vorderes Nadelbett und ein hinteres Nadelbett enthält, wobei das Grundgestrick (3) aus einer auf dem vorderen Nadelbett der Flachstrickmaschine gestrickten vorderen Lage (v) und einer der vorderen Lage (v) gegenüberliegenden hinteren Lage (h), die auf dem hinteren Nadelbett gestrickt ist, gebildet wird.

10. Verfahren nach Anspruch 9, **dadurch gekennzeichnet, dass** die vordere Lage (v) und die hintere Lage (h) jeweils den Strickfaden (4) enthalten, wobei der Strickfaden (4) zur Ausbildung der rippenförmigen Erhebungen (2) Flottungen (F) oder Fanghenkel (f) bildet und die Stellen, an denen der Strickfaden (4) flott liegt oder Fanghenkel (f) bildet, in jeweils aufeinanderfolgenden Maschenreihen (m) um wenigstens eine Masche, bevorzugt um eine oder zwei Maschen, versetzt zueinander angeordnet werden.

11. Verfahren nach einem der voranstehenden Ansprüche, **dadurch gekennzeichnet, dass** es sich bei dem elastischen Schussfaden (5) um ein Umwindegarn mit einem hochelastischen Kernfaden, der bevorzugt eine Dicke im Bereich von 200 bis 1500 dtex aufweist, oder um einen Elasthan- oder Gummifaden handelt.

12. Verfahren nach einem der voranstehenden Ansprüche, **dadurch gekennzeichnet, dass** das Grundgestrick (3) als Rechts-Links-Gestrick und/oder in einer 1:1-Bindung gestrickt wird.

13. Verfahren nach einem der voranstehenden Ansprüche, **dadurch gekennzeichnet, dass** es sich bei dem Kompressionsartikel um einen Handschuh handelt, wobei eine Aufnahme (1) zur Aufnahme eines Daumens und/oder wenigstens eine weitere Aufnahme (1a, 1b, 1c, 1d) zur Aufnahme eines Fingers gestrickt wird, oder dass es sich bei dem Kompressionsartikel um einen Strumpf handelt, wobei wenigstens eine Aufnahme (1) zur Aufnahme einer Zehe und/oder weitere Aufnahmen (1a, 1b, 1c, 1d) zur Aufnahme weiterer Zehen gestrickt werden.

14. Verfahren nach einem der voranstehenden Ansprüche, wobei in das Grundgestrick (3) ein vollflächig durchgehendes Rippenmuster und zusätzlich zumindest im Bereich der oder jeder Aufnahme (1) die rippenförmigen Erhebungen (2) so eingestrickt werden, dass die rippenförmigen Erhebungen (2) über dem Rippenmuster des Grundgestricks (3) vorstehen.

## Claims

1. Method for producing a seamless compression article on a flat knitting machine with a front needle bed and a rear needle bed opposite thereto, wherein a basic knitted fabric (3) is knitted on the flat knitting machine from at least one knitting yarn (4) and at least one elastic weft yarn (5) inserted or knitted therein, wherein the basic knitted fabric (3) has an inner side (I) facing the body extremity when worn and an outer side (A) opposite thereto and the basic knitted fabric (3) is knitted along a wale direction (s) extending at least substantially in the longitudinal direction (L) of the or of each receptacle (1) and has wale rows (m) extending transversely to the wale direction (s), and the compression article knitted from the basic knitted fabric (3) has at least one tubular or pocket-shaped receptacle (1) extending along a longitudinal direction (L) for at least one finger (F) or at least one toe (Z) of a wearer of the compression article, so that the compression article can be placed on a body extremity of the wearer extending in the longitudinal direction (L), wherein a plurality of rib-shaped protrusions (2), which extend at least substantially parallel to one another and along the longitudinal direction (L) of the respective receptacle (1), is knitted into each receptacle (1), wherein the rib-shaped protrusions (2) protrude on the outside (A) above the basic knitted fabric (3), **characterized in that** the rib-shaped protrusions (2) do not appear on the inside (I).

2. The method according to claim 1, **characterized in that** between two and 30, preferably between three and seven, protrusions (2) are arranged on each receptacle (1) and/or **in that** the protrusions (2) extend over at least two, preferably over three or four, stitches in a wale row (m) of the basic knitted fabric (3).

3. The method according to any one of the preceding claims, **characterized in that** adjacent protrusions (2) are knitted in parallel and at a predetermined distance (d) from one another, wherein preferably at least two stitches of the knitting yarn (4), preferably between three and 20 stitches, particularly preferably between five and ten stitches of the knitting yarn (4), are lying between two successive protrusions (2) in a wale row (m).

4. The method according to any one of the preceding claims, **characterized in that** a weft yarn (5) is knitted or bound into each or every second wale row (m) of the basic knitted fabric (3), and **in that** optionally a plurality of wale rows (m) are knitted in succession in the longitudinal direction (L) in pressure-reduced regions of the basic knitted fabric (3), in which no weft yarn is running.

5. The method according to any one of the preceding claims, **characterized in that** the weft yarn (5) is bound into the basic knitted fabric (3) of the knitting yarn (4) as a tuck stitch (f) in every or every second stitch between two successive protrusions (2) in the direction of the wale rows (m).

6. The method according to any one of the preceding claims, **characterized in that** the weft yarn (5) forms two tuck stitches (f-f) following one another in a wale row (m) in an edge region of the protrusions (2).

7. The method according to any one of the preceding claims, **characterized in that** the protrusions (2) extend in the longitudinal direction (L) of the or each receptacle (1) at least substantially over the entire extent of the respective receptacle (1), preferably with no protrusions (2) being knitted in the regions of the compression article outside the receptacles (1).

8. The method according to any one of the preceding claims, **characterized in that** the rib-shaped protrusions (2) are formed by floats (F) and/or tuck stitches (f) of the knitting yarn (3).

9. The method according to any one of the preceding claims, **characterized in that** the flat knitting machine comprises a front needle bed and a rear needle bed, the basic knitted fabric (3) being formed from a front layer (v) knitted on the front needle bed of the flat knitting machine and a rear layer (h) opposite the front layer (v) and knitted on the rear needle bed.

10. The method according to claim 9, **characterized in that** the front layer (v) and the rear layer (h) each contain the knitting yarn (4), with the knitting yarn (4) forming floats (F) or tuck stitches (f) to form the rib-shaped protrusions (2), and the points at which the knitting yarn (4) lies floating or forms tuck stitches (f) being arranged offset from one another by at least one stitch, preferably by one or two stitches, in successive wale rows (m).

11. The method according to any one of the preceding claims, **characterized in that** the elastic weft yarn (5) is a covered yarn with a highly elastic core yarn, which preferably has a thickness in the range from 200 to 1500 dtex, or is an elastane or rubber yarn.

12. The method according to any one of the preceding claims, **characterized in that** the basic knitted fabric (3) is knitted as a right-to-left knitted fabric and/or the basic knitted fabric (3) is knitted in a 1:1 weave.

13. The method according to any one of the preceding claims, **characterized in that** the compression article is a glove, wherein one receptacle (1) is knitted to accommodate a thumb and/or at least one further receptacle (1a, 1b, 1c, 1d) is knitted to accommodate a finger, or **in that** the compression article is a stocking, wherein at least one receptacle (1) is knitted to accommodate a toe and/or further receptacles (1a, 1b, 1c, Id) are knitted to accommodate further toes.

14. The method according to any one of the preceding claims, wherein a full-surface continuous rib pattern and additionally, at least in the region of the or each receptacle (1), the rib-shaped protrusions (2) are knitted into the basic knitted fabric (3) in such a way that the rib-shaped protrusions (2) protrude above the rib pattern of the basic knitted fabric (3).

## Revendications

1. Procédé de fabrication d'un article de contention sans couture sur un métier à tricoter rectiligne avec un lit d'aiguilles avant et un lit d'aiguilles arrière opposé à celui-ci, dans lequel un tricot de base (3) consistant en au moins un fil à tricoter (4) et au moins un fil de trame (5) élastique qui est inséré ou tricoté dans celui-ci est tricoté sur le métier à tricoter, dans lequel le tricot de base (3) présente un côté intérieur (I) tourné vers l'extrémité de corps quand il est porté et un côté extérieur (A) se situant à l'opposé de celui-ci et le tricot de base (3) est tricoté dans une direction de colonne de mailles (s) s'étendant au moins essentiellement dans la direction longitudinale (L) du ou de chaque logement (1) et présente des rangées de mailles (m) se déroulant transversalement à la direction de colonne de mailles (s), et l'article de contention tricoté à partir du tricot de base (3) présente au moins un logement (1) en forme de tube ou de poche et s'étendant dans une direction longitudinale (L) pour au moins un doigt (F) ou au moins un orteil (Z) d'un porteur de l'article de contention, de sorte que l'article de contention puisse être appliqué contre une extrémité de corps du porteur s'étendant dans la direction longitudinale (L), dans lequel une pluralité d'élévations (2) en forme de nervure, qui se déroulent au moins essentiellement parallèlement les unes aux autres et dans la direction longitudinale (L) du logement (1) respectif, est tricotée dans chaque logement (1), dans lequel les élévations (2) en forme de nervure font saillie sur le côté extérieur (A) par-dessus le tricot de base (3), **caractérisé en ce que** les élévations (2) en forme de nervure n'apparaissent pas sur le côté intérieur (I).

2. Procédé selon la revendication 1, **caractérisé en ce qu'**entre deux et 30, de préférence entre trois et sept élévations (2) sont agencées contre chaque logement (1) et/ou **en ce que** les élévations (2) s'étendent dans une rangée de mailles (m) du tricot de base (3) par-dessus au moins deux, de préférence par-dessus trois ou quatre mailles.

3. Procédé selon l'une des revendications précédentes, **caractérisé en ce que** des élévations (2) avoisinantes sont tricotées de façon à se dérouler parallèlement et à une distance (d) prédéfinie les unes par rapport aux autres, dans lequel au moins deux mailles du fil à tricoter (4), de préférence entre trois et 20 mailles, particulièrement de préférence entre cinq et dix mailles du fil à tricoter (4) se situent de préférence entre deux élévations (2) se succédant dans une rangée de mailles (m).

4. Procédé selon l'une des revendications précédentes, **caractérisé en ce qu'**un fil de trame (5) est tricoté ou intégré dans chaque rangée de mailles (m) ou dans une rangée de mailles sur deux du tricot de base (3) et **en ce qu'**optionnellement dans des zones diminuées par pression du tricot de base (3) sont tricotées plusieurs rangées de mailles (m) se succédant dans la direction longitudinale (L), dans lesquelles aucun fil de trame ne se déroule.

5. Procédé selon l'une des revendications précédentes, **caractérisé en ce que** le fil de trame (5) est intégré dans le tricot de base (3) du fil à tricoter (4) en tant que maille chargée (f), entre deux élévations (2) se succédant dans la direction des rangées de mailles (m), dans chaque maille ou dans une maille sur deux.

6. Procédé selon l'une des revendications précédentes, **caractérisé en ce que** dans une zone de bord des élévations (2) le fil de trame (5) forme deux mailles chargées (f-f) se succédant dans une rangée de mailles (m).

7. Procédé selon l'une des revendications précédentes, **caractérisé en ce que** dans la direction longitudinale (L) du ou de chaque logement (1) les élévations (2) s'étendent au moins essentiellement sur l'ensemble de l'extension du logement (1) respectif, dans lequel de préférence dans les zones de l'article de contention aucune élévation (2) n'est tricotée à l'extérieur des logements (1).

8. Procédé selon l'une des revendications précédentes, **caractérisé en ce que** les élévations (2) en forme de nervure sont formées par des mailles flottées (F) et/ou des mailles chargées (f) du fil à tricoter (3).

9. Procédé selon l'une des revendications précédentes, **caractérisé en ce que** le métier à tricoter rectiligne contient un lit d'aiguilles avant et un lit d'aiguilles arrière, dans lequel le tricot de base (3) est formé par une couche avant (v) tricotée sur le lit d'aiguilles avant du métier à tricoter rectiligne et par une couche arrière (h) opposée à la couche avant (v), qui est tricotée sur le lit d'aiguilles arrière.

10. Procédé selon la revendication 9, **caractérisé en ce que** la couche avant (v) et la couche arrière (h) contiennent respectivement le fil à tricoter (4), dans lequel le fil à tricoter (4) forme des mailles flottées (F) ou des boucles de charge (f) pour la formation des élévations (2) en forme de nervure, et les emplacements contre lesquels le fils à tricoter (4) se situe en flottant ou forme des boucles de charge (f) sont agencés de façon décalée les uns par rapport aux autres d'au moins une maille, de préférence d'une ou deux mailles, dans des rangées de mailles (m) se succédant respectivement.

11. Procédé selon l'une des revendications précédentes, **caractérisé en ce que** le fil de trame (5) élastique est un fil guipé avec une âme hautement élastique qui présente de préférence une épaisseur dans la plage de 200 à 1 500 dtex, ou un fil d'élasthanne ou de caoutchouc.

12. Procédé selon l'une des revendications précédentes, **caractérisé en ce que** le tricot de base (3) est tricoté en tant que tricot droite-gauche et/ou dans une armure 1/1.

13. Procédé selon l'une des revendications précédentes, **caractérisé en ce que** l'article de contention est un gant, dans lequel un logement (1) est tricoté pour le logement d'un pouce et/ou d'au moins un autre logement (1a, 1b, 1c, 1d) pour le logement d'un doigt, ou **en ce que** l'article de contention est un bas, dans lequel au moins un logement (1) est tricoté pour le logement d'un orteil et/ou d'autres logements (1a, 1b, 1c, 1d) pour le logement d'autres orteils.

14. Procédé selon l'une des revendications précédentes, dans lequel un motif de nervure continu sur toute la surface est tricoté dans le tricot de base (3) et en complément au moins dans la zone du ou de chaque logement (1) sont tricotées les élévations (2) en forme de nervure, de sorte que les élévations (2) en forme de nervure fassent saillie par-dessus le motif de nervure du tricot de base (3).
